# EUROPÄISCHE PATENTSCHRIFT

(11) **EP 4 252 809 B1**
(45) Veröffentlichungstag und Bekanntmachung des Hinweises auf die Patenterteilung: **26.03.2025**
(21) Anmeldenummer: 23150445.7
(22) Anmeldetag: 05.01.2023
(51) Int. Cl.: A61M 16/08

(54) **VERFAHREN ZUR HERSTELLUNG EINER BAUGRUPPE FÜR EIN PATIENTEN-BEATMUNGSSYSTEM**
METHOD FOR PRODUCING AN ASSEMBLY FOR A PATIENT VENTILATION SYSTEM
PROCÉDÉ DE FABRICATION D'UN ENSEMBLE POUR UN SYSTÈME DE VENTILATION DE PATIENT

(30) Priorität: 28.03.2022 DE 102022203012
(43) Veröffentlichungstag der Anmeldung: 04.10.2023
(73) Patentinhaber: New Ventures GmbH, 95111 Rehau (DE); Raumedic AG, 95213 Münchberg (DE)
(72) Erfinder: Ehrenpfordt, Ricardo, 08056 Zwickau (DE); Ranfeld, Constanze, 95111 Rehau (DE); Golbs, Lydia, 95182 Döhlau (DE); Ruhland, Thomas, 95444 Bayreuth (DE)
(74) Vertreter: Schmitt-Nilson Schraud Waibel Wohlfrom Patentanwälte Partnerschaft mbB

(56) Entgegenhaltungen:
- WO-A1-2020/089001
- DE-A1- 102020 212 441
- DE-U1- 202005 008 156
- US-A1- 2016 199 612

## Beschreibung

Die Erfindung betrifft ein Verfahren zur Herstellung einer Baugruppe für ein Patienten-Beatmungssystem.

Komponenten für ein Patienten-Beatmungssystem sind bekannt aus der US 2017/0 197 055 Al, der WO 2007/051 230 Al, der DE 10 2019 216 489 A1, der US 9,903,371 B2 und der US 2019/0 290 866 A1. Vom Markt her ist weiterhin ein Patienten-Beatmungssystem "VentStar Helix" bekannt. Die DE 10 2018 218 629 A1 offenbart ein Verfahren zur Herstellung eines abgewinkelten Steckverbinders.

In der US 2016/199612 A1 wird eine Verbindungsanordnung für ein Atemtherapiesystem beschrieben, die umfasst: eine Auslassbaugruppe, wobei die Auslassbaugruppe ein Auslassgehäuse und eine an dem Auslassgehäuse angeordnete Drehscheibe umfasst, wobei das Auslassgehäuse und die Drehscheibe zumindest teilweise eine Aussparung definieren; einen Auslassverbinder, der an einem Ende eines Schlauchabschnitts angeordnet ist, wobei der Auslassverbinder einen elektrischen Verbinder umfasst; und ein Kabel mit einem ersten Ende zum Verbinden mit dem elektrischen Verbinder und einem zweiten Ende zum Verbinden mit mindestens einer elektrischen Komponente des Atemtherapiesystems, wobei das Kabel einen schlaffen Abschnitt aufweist, wobei der Auslassverbinder und die Drehscheibe gemeinsam zwischen einer ersten Position und einer zweiten Position drehbar sind, und wobei sich der schlaffe Abschnitt des Kabels aus der Aussparung erstreckt und sich um die Drehscheibe wickelt, wenn die Drehscheibe von der ersten Position in die zweite Position gedreht wird.

Es ist eine Aufgabe der vorliegenden Erfindung, ein Fertigungsverfahren für eine Baugruppe eines derartigen Patienten-Beatmungssystems derart weiterzubilden, dass es sich für die Massenproduktion eignet.

Die Aufgabe ist erfindungsgemäß gelöst durch ein Verfahren mit den im Anspruch 1 angegebenen Merkmalen.

Erfindungsgemäß wurde erkannt, dass es möglich ist, die Baugruppe mit einer Fertigungssequenz herzustellen, die zwei Umspritzschritte beinhaltet. Ein erster Umspritzschritt dient dabei zur Fixierung einer vorbereiteten Leitungsverbindung, über die insbesondere eine elektrische Verbindung zwischen Mantel-Drähten des Atemluft-Schlauchabschnitts und der Elektronikkomponente hergestellt werden kann. Die Kontaktpins der Komponentenaufnahme können als Endabschnitte eines jeweiligen Leiterträgers der Leitungsverbindung ausgeführt sein. Die Leitungsträger-Gehäusehülse des Konnektors als Ergebnis des ersten Umspritzschritts hat mindestens einen Hülsenabschnitt zur Führung der Atemluft. Im nachgelagerten weiteren Umspritzschritt wird dann zusammen mit einem äußeren Konnektorgehäuse eine Komponentenaufnahme für die Elektronikkomponente geformt. Die Elektronikkomponente kann dann wahlweise in der Komponentenaufnahme aufgenommen sein oder es kann dort ein Blind-Deckel eingesetzt sein. Bei der Elektronikkomponente kann es sich um eine Sensoreinrichtung handeln. Neben Atemluft-Parametern (Temperatur, Feuchtigkeit, Atemdruck, Atemluft-Zusammensetzung) können mit der Sensoreinrichtung, die mindestens einen derartigen Sensor aufweist, auch weitere Parameter, nämlich Parameter des Patienten, die der Beatmungstechnik und/oder Parameter der Umgebung vermessen werden.

Mit dem Temperatursensor ist insbesondere eine Temperaturwert-Schwellwerterkennung möglich. Dies kann zu medizinischen Zwecken, aber auch zu anderen Zwecken eingesetzt werden, wie beispielsweise zum Brandschutz. Eine Genauigkeit des Temperatursensors kann besser sein als 0,5 K und kann beispielsweise bei 0,2 K, bei 0,1 K oder auch bei 0,05 K liegen. Diese Genauigkeit kann in einem Bereich zwischen -10°C und 80°C gewährleistet sein. Auch ein engerer Temperaturbereich, in dem die Genauigkeit gewährleistet ist, ist möglich, beispielsweise zwischen 0°C und 50°C oder zwischen 10°C und 50°C.

Soweit ein Temperatursensor als Elektronikkomponente zum Einsatz kommt, ist mit diesem beispielsweise eine Temperaturwert-Schwellwerterkennung möglich.

Bei der weiteren, die Beatmungsluft führenden Komponente des Patienten-Beatmungssystems, zu der der Konnektor der Baugruppe eine Verbindung herstellt, kann es sich um ein Patienten-Interface handeln. Alternativ kann es sich bei dieser weiteren Komponente z. B. um einen Befeuchter handeln.

Ein Vorformen nach Anspruch 1 hat sich als für den Massenproduktionseinsatz besonders geeignet herausgestellt. Bei der 2D-Leiterstruktur, von der ausgehend die Leitungsverbindung bereitgestellt wird, kann es sich um einen Leadframe mit einer Vielzahl entsprechend vorgefertigter Leitungskomponenten handeln. Bei der 2D-Leiterstruktur kann es sich um ein Stanzgitter handeln.

Die 2D-Leiterstruktur kann aus Metall und insbesondere aus Kupfer gefertigt sein. Alternativ kann es sich bei der 2D-Leiterstruktur auch um einen duroplastischen Leitungsträger, zum Beispiel aus PCB, und/oder um eine Leiterplatte handeln.

Die 2D-Leiterstruktur kann eine Rahmen-Trägerkomponente aufweisen, die die jeweiligen Leitungsverbindungskomponenten trägt. Komponenten, die für eine jeweils ausgewählte Leitungsverbindung nicht genutzt werden, können dann Bestandteil der Rahmen-Trägerkomponente sein. Derartige nicht für die elektrische Übertragung genutzte Komponenten können auch zunächst als Haltekomponenten bei der bereitgestellten Leitungsverbindung verbleiben und später und gegebenenfalls auch nach dem elektrischen Kontaktieren noch entfernt werden.

Die Vorformung der 3D-Leitungsverbindung nach Anspruch 2 erfolgt so, dass die resultierende Leitungsverbindung sich nicht flächig erstreckt, sondern in allen drei Raumdimensionen eine Erstreckung aufweist, die über den Leitungsquerschnitt von Leitungskomponenten der Leitungsverbindung hinausgeht.

Ein Umspritzen in einem als Mehrfachkavität ausgeführten Spritzgusswerkzeug nach Anspruch 1 erhöht einen Fertigungsdurchsatz des Herstellungsverfahrens. Die vereinzelten inneren Leitungsträger-Gehäusehülsen, die nach diesem Mehrfachkavitäts-Umspritzen resultieren, können dann einzeln weiterverarbeitet und insbesondere einzeln weiter zur Formung des äußeren Konnektorgehäuses umspritzt werden.

Ultraschall-Schweißen nach Anspruch 3 hat sich als zum elektrischen Verbinden besonders geeignet herausgestellt.

Ein Ausführungsbeispiel der Erfindung wird nachfolgend anhand der Zeichnung näher erläutert. In dieser zeigen:

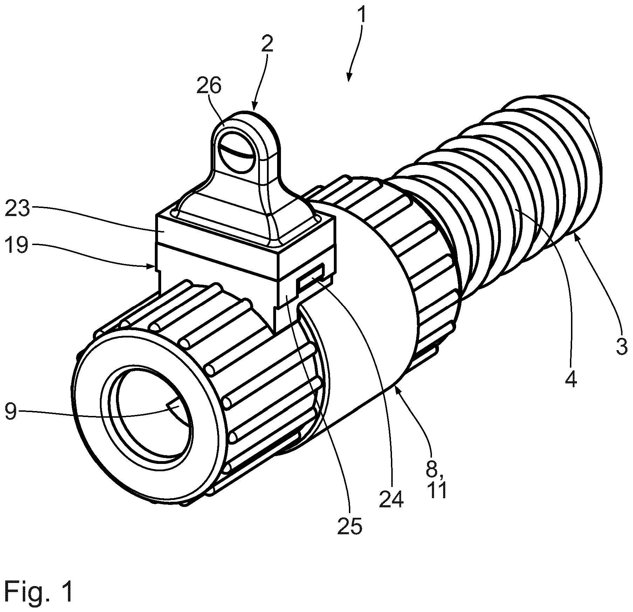

- Figur 1: perspektivisch eine Baugruppe für ein Patienten-Beatmungssystem mit einem Atemluft-Schlauchabschnitt, einem hiermit verbundenen Konnektor zum Verbinden des Atemluft-Schlauchabschnitts mit einem Patienten-Interface und einer Sensoraufnahme, in die eine Temperatursensoreinrichtung eingesetzt ist;

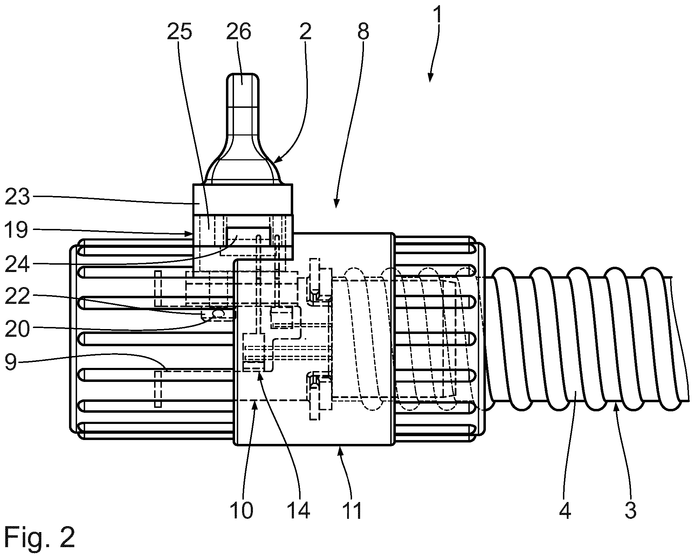

- Figur 2: eine innere Details preisgebende Seitenansicht der Baugruppe nach Figur 1, wiederum mit eingesteckter Temperatursensoreinrichtung;

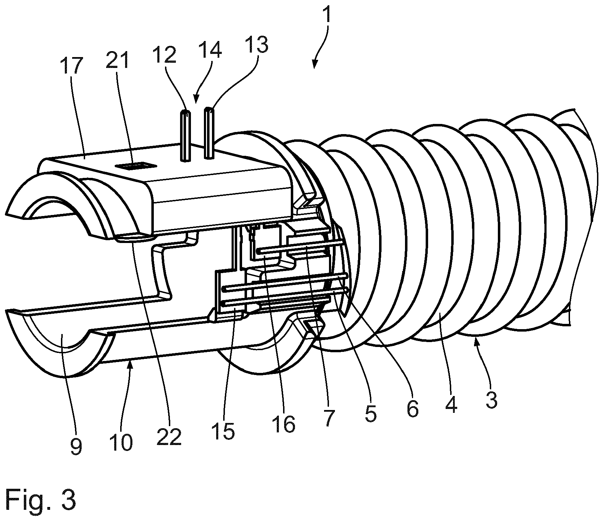

- Figur 3: wiederum perspektivisch die Baugruppe in einem Zwischenstadium ihrer Herstellung, wobei eine vorgeformte 3D-Lei- tungsverbindung jeweils einteilig mit Kontaktpins der Sensoraufnahme und Mantel-Drähten des Atemluft-Schlauchabschnitts zur Formung einer inneren Leitungsträger-Gehäusehülse des Konnektors umspritzt sind;

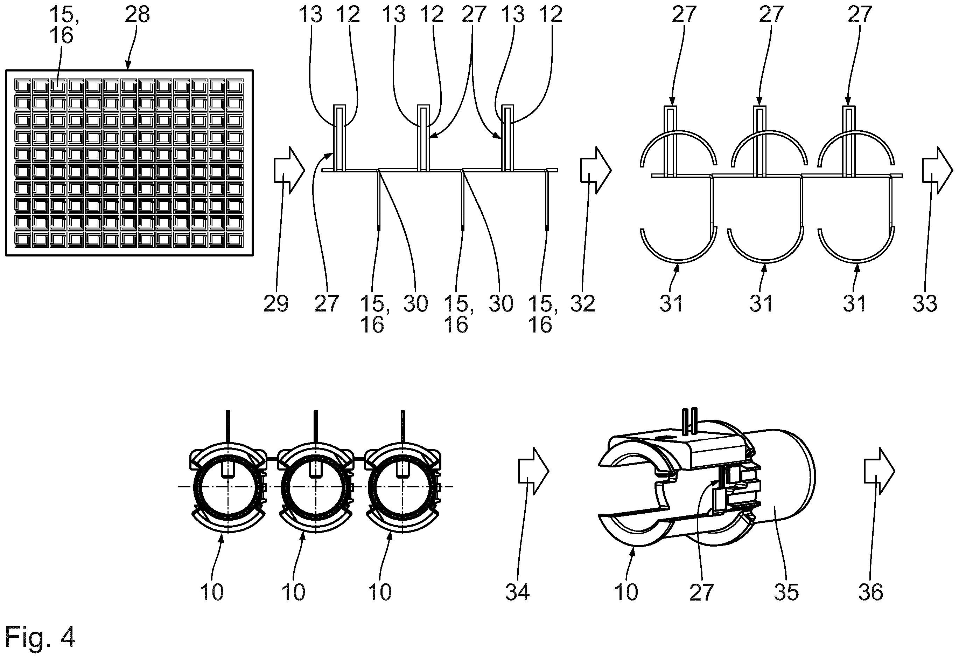

- Figur 4: teilweise schematisch Verarbeitungsschritte bei einem Verfahren zur Herstellung der Baugruppe einschließlich der Herstellung mehrerer innerer Leitungsträger-Gehäusehülsen durch Umspritzen und nachfolgend mechanische Trennung zunächst miteinander verbundener 3D-Leitungsverbindungen, die beim Umspritzschritt innerhalb einer Mehrfachkavität noch miteinander verbunden waren;

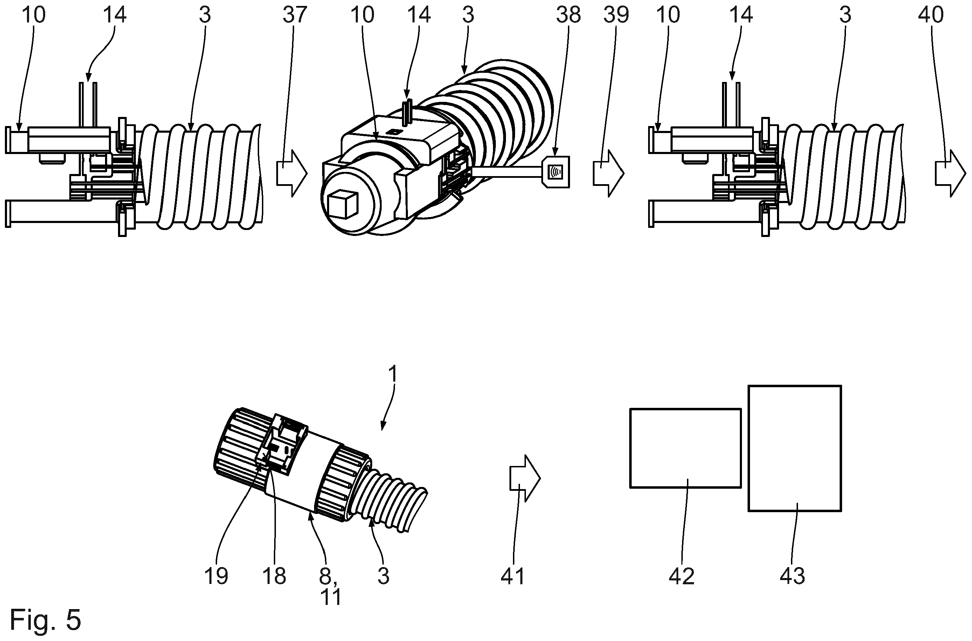

- Figur 5: weitere Verarbeitungsschritte des Herstellungsverfahrens bis zur Herstellung der fertigen Baugruppe und einer sich anschließenden Qualitätskontrolle;

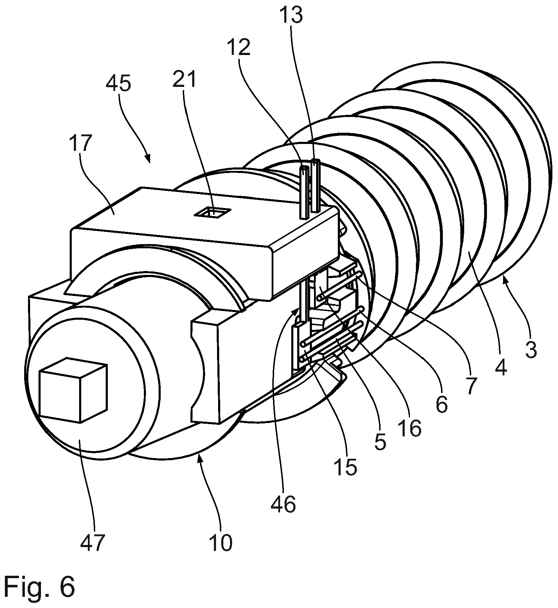

- Figur 6: in einer zu Figur 3 ähnlichen Darstellung, die zu dem einen Kern eines Ultraschallschweißwerkzeuges zeigt, eine weitere Ausführung der Baugruppe im Zwischenstadium ihrer Herstellung, wobei eine vorbereitete Leitungsverbindung, ausgeführt als 2D-Leitungsverbindung mit den Kontaktpins der Sensoraufnahme und den Mantel-Drähten des Atemluft-Schlauchabschnitts zur Formung der inneren Leitungsträger-Gehäusehülse des Konnektors umspritzt ist;

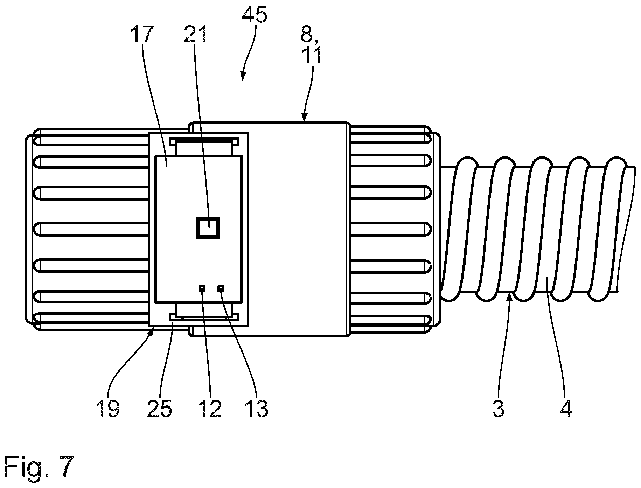

- Figur 7: eine Aufsicht auf die komplett hergestellte Baugruppe, ausgehend vom Zwischenstadium nach Figur 6, wobei die Baugruppe ohne eingesetzte Temperatursensoreinrichtung dargestellt ist.

Ein Patienten-Beatmungssystem, zu dem eine Baugruppe 1 gehört, die mit einer eingesteckten Temperatursensoreinrichtung 2 in der Figur 1 perspektivisch dargestellt ist, dient zur Beatmung eines Patienten im klinischen, sonstigen stationären oder auch häuslichen Pflegebereich. Grundsätzlich ist ein derartiges Patienten-Beatmungssystem bekannt aus der WO 2007/051 230 A1 und der DE 10 2019 216 489 A1. Vom Markt her ist ein entsprechendes Patienten-Beatmungssystem bekannt vom Produkt "VentStar Helix".

Die wesentlichen, luftführenden Komponenten des Beatmungssystems 1 sind aus Kunststoff.

Die Baugruppe 1 hat einen Atemluft-Schlauchabschnitt 3 zur Führung von Beatmungsluft von einer nicht dargestellten Atemluftquelle hin zu einem Patienten.

Längs eines Schlauchmantels 4 des Atemluft-Schlauchabschnitts 3 sind elektrisch leitfähige Mantel-Drähte 5, 6 und 7 (vgl. Figur 3) geführt. Diese Führung erfolgt wendelförmig um ein inneres Luftführungslumen des Atemluft-Schlauchabschnitts 3. Die Mantel-Drähte 5 bis 7 sind hierzu wendelförmig mit Material des Atemluft-Schlauchabschnitts umspritzt. Zwei dieser Mantel-Drähte, nämlich die Mantel-Drähte 5 und 6 stellen Heizdrähte für den Atemluft-Schlauchabschnitt 3 dar, die über eine nicht dargestellte Versorgungseinrichtung mit Heizstrom versorgt werden können. Der dritte Mantel-Draht 7 stellt eine Signalleitung für die Temperatursensoreinrichtung 2 dar.

Mit dem Atemluft-Schlauchabschnitt 3 ist ein Konnektor 8 der Baugruppe 1 durch Umspritzen des Atemluft-Schlauchabschnitts 3 verbunden. Der Konnektor 8 dient zum Verbinden des Atemluft-Schlauchabschnitts 3 mit einem in der Zeichnung nicht dargestellten Patienten-Interface des Patienten-Beatmungssystems. Diese Verbindung ist so, dass eine innere Luftführungs-Konnektorkomponente 9 des Konnektors 8 jedenfalls abschnittsweise einen integralen Bestandteil mit dem Atemluft-Schlauchabschnitt 3 bildet.

Der Konnektor 8 hat eine innere Leitungsträger-Gehäusehülse 10 und ein äußeres Konnektorgehäuse 11. Diejenigen Abschnitte der Luftführungs-Konnektorkomponente 9, die an den Atemluft-Schlauchabschnitt 3 angeformt und somit einen integralen Bestandteil mit diesem bilden, sind Abschnitte des äußeren Konnektorgehäuses 11 des Konnektors 8.

Eingeformt in die innere Leitungsträger-Gehäusehülse 10 sind Leitungskomponenten 12, 13 einer sich dreidimensional erstreckenden 3D-Leitungsverbindung der Baugruppe 1. Diese 3D-Leitungsverbindung wird nachfolgend noch erläutert.

Die Leitungskomponente 12 ist über ein Kontaktpad 15 mit den beiden Heiz-Mantel-Drähten 5, 6 elektrisch verbunden und stellt eine Kurzschlussbrücke für einen Heizkreis des Atemluft-Schlauchabschnitts 3 dar. Die Leitungskomponente 13 ist über ein Kontaktpad 16 elektrisch mit den Signal-Mantel-Draht 7 verbunden. Zum Kontaktieren mit den Kontaktpads 15, 16 sind die Mantel-Drähte 5 bis 7 aus der wendelförmigen Umspritzung freigelegt. Von den Kontaktpads 15, 16 abgewandt enden die Leitungskomponenten 12, 13 in Kontaktpins, die über eine isolierende Verbindungsplatte 17 der inneren Leitungsträger-Gehäusehülse 10 überstehen. Bei eingesteckter Temperatursensoreinrichtung 2 sind die Kontaktpins der Leitungskomponenten 12, 13 mit entsprechenden Kontaktbuchsen der Temperatursensoreinrichtung 2 elektrisch verbunden.

Die beiden überstehen Kontaktpins der Leitungskomponenten 12, 13 dienen zum elektrischen Konnektieren der Mantel-Drähte 5 bis 7 mit der Temperatursensoreinrichtung 2.

Zusammen mit einem Aufnahmekörper 18 (vgl. die vorletzte Abbildung der Figur 5), der Bestandteil des äußeren Konnektorgehäuses 11 ist, stellt die Verbindungsplatte 17 eine Sensoraufnahme 19 zum Einsetzen der Temperatursensoreinrichtung 2 in den Konnektor 8 und insbesondere zum Einsetzen eines Temperatursensors 20 der Temperatursensoreinrichtung 2 in die Luftführungs-Konnektorkomponente 9, also in dessen Luftführungslumen, dar. Die Sensoraufnahme 19 ist einstückig in das äußere Konnektorgehäuse 11 des Konnektors 8 eingeformt. Der Temperatursensor 20 dient zum Messen einer Temperatur der durch die Luftführungs-Konnektorkomponente 9 strömenden Beatmungsluft dar.

Die Temperatursensoreinrichtung 2 ist ein Beispiel für eine in die Aufnahme 19 einsteckbare Elektronikkomponente. Die Sensoraufnahme 19 ist daher allgemeiner eine Komponentenaufnahme. Anstelle eines Temperatursensors kann bei der Elektronikkomponente auch eine Sensoreinrichtung zur Erfassung eines weiteren Parameters, insbesondere eines Atemluft-Parameters, zum Einsatz kommen.

Bei der Sensoreinrichtung kann es sich einen Sensor zur Messung der Luftfeuchtigkeit oder auch um einen chemischen oder spektroskopischen Sensor handeln. Auch eine andere Elektronikkomponente kann in die Komponentenaufnahme 19 eingesetzt werden, beispielsweise eine Überwachungseinheit beispielsweise zur Bestimmung einer Gebrauchsdauer oder einer Gebrauchsbelastung der Baugruppe 1.

Die Sensoraufnahme 19 hat eine Durchführung 21 zum Durchführen des Temperatursensors 20 der Temperatursensoreinrichtung 2 in das Luftführungslumen der Luftführungs-Konnektorkomponente 9. Diese Durchführung 21 ist als Öffnung in der Verbindungsplatte 17 ausgeführt, die in das Luftführungslumen der Luftführungs-Konnektorkomponente 9 einmündet.

Zudem hat die Sensoraufnahme 19 eine Sensor-Führungskomponente 22, die unter anderem zur Führung einer Einsetzbewegung des Temperatursensors 20 in das Luftführungslumen der Luftführungs-Konnektorkomponente 9 dient. Die Sensor-Führungskomponente 22 stellt zudem einen Strömungs- und/oder Berührungsschutz für den Temperatursensor 20 dar.

Die Sensor-Führungskomponente 22 kann mindestens ein Fenster aufweisen, über das das Luftführungslumen vom Temperatursensor 20 her zur Temperaturmessung der im Luftführungslumen geführten Beatmungsluft zugänglich ist. Die Sensor-Führungskomponente 22 kann thermisch-leitfähige Komponenten enthalten zur thermischen Anbindung des Temperatursensors 20 an das Luftführungslumen.

Die Sensoraufnahme 19 ist zum steckbaren Einsetzen der Temperatursensoreinrichtung 2 ausgeführt. Hierzu hat die Temperatursensoreinrichtung 2 einen Tragkörper 23, über den der in die Sensoraufnahme 19 eingesteckte Temperatursensor 20 nach unten in das Luftführungslumen der Luftführungs-Konnektorkomponente 9 übersteht. An den Tragkörper 23 ist ein Rastabschnitt 24 (vgl. Figur 1) zum Verrasten mit einem Gegen-Rastabschnitt 25 der Sensoraufnahme 19 angeformt. Der Gegen-Rastabschnitt 25 wiederum ist angeformter Bestandteil der Sensoraufnahme 19.

Im Tragkörper 23 sind Kontaktpins der Temperatursensoreinrichtung zur Verbindung mit den Kontaktpins der Leitungskomponenten 12, 13 vorgesehen.

Ebenfalls an den Tragkörper 23 angeformt ist ein Griffabschnitt 26 der Temperatursensoreinrichtung 2 zum Erfassen eines vom Temperatursensor 20 abgewandten Abschnitts des Tragkörpers 23.

Ein Verfahren zum Herstellen der Baugruppe 1 wird nachfolgend anhand der Figuren 4 und 5 näher erläutert.

Zum Herstellen der Baugruppe 1 wird zunächst eine sich dreidimensional erstreckende Leitungsverbindung 27 ausgehend von einer als Leadframe ausgeführten 2D-Leiterstruktur 28 vorgeformt. Die Leitungskomponenten der 2D-Leitungsstruktur 28 können Querschnitte ausweisen, die kleiner sind als 0,5 mm und die im Bereich zwischen 50 µm und 200 µm liegen können.

Die jeweilige 3D-Leitungsverbindung 27 erstreckt sich zwischen den jeweiligen Kontaktpads 15, 16 und den Kontaktpins der Leitungskomponenten 12, 13. In der 2D-Leiterstruktur 28 sind eine Vielzahl von Kontaktpads 15 beziehungsweise 16 über entsprechende Leiterbahnen miteinander verbunden, die im Rahmen eines Vorformschritts 29 gegeneinander insbesondere um 90° zur 3D-Leitungsverbindung 27 aufgebogen werden.

Ergebnis des Vorformschritts 29 ist eine Mehrzahl von 3D-Leitungsverbindungen 27, im vorliegenden Beispiel 3 derartige 3D-Leitungsverbindungen 27, die einer entsprechenden Mehrzahl später anzuformender innerer Leitungsträger-Gehäusehülsen 10 eines jeweiligen Konnektors 8 zugeordnet sind. Diese mehreren 3D-Leitungsverbindungen 27 sind nach dem Vorformschritt 29 zunächst noch mechanisch über Sollbruchstellen 30 miteinander verbunden.

Nach dem Vorformen 29 der 3D-Leitungsverbindungen 27 werden die noch mechanisch miteinander verbundenen 3D-Leitungsverbindungen 27 in ein Spritzgusswerkzeug in Form einer Spritzguss-Mehrfachkavität 31 eingesetzt, was in einem Einsetzschritt 32 erfolgt. In Anschluss hieran geschieht in einem Umspritzschritt 33 ein Umspritzen der 3D-Leitungsverbindungen 27 zur Formung der inneren Leitungsträger-Gehäusehülsen 10 des jeweiligen Konnektors 8. Beim Umspritzschritt 33 wird gleichzeitig die noch mechanisch miteinander verbundene Mehrzahl der 3D-Leitungsverbindungen 27 zur Formung der entsprechenden Mehrzahl innerer Leitungsträger-Gehäusehülsen 10 in der Spritzguss-Mehrfachkavität 31 umspritzt.

Ergebnis des Umspritzschritts 33 ist insbesondere die Ausformung der inneren Leitungsträger-Gehäusehülse 10 mit mindestens einem Hülsenabschnitt, der der Führung der Atemluft dient.

Nach dem Umspritzschritt 33 erfolgt in einem Trennschritt 34 eine mechanische Trennung der miteinander verbundenen 3D-Leitungsverbindungen 27 an den Sollbruchstellen 30 und damit eine Vereinzelung der inneren Leitungsträger-Gehäusehülsen 10.

Im Anschluss hieran wird eine Anschlusshülse 35 der vereinzelten inneren Leitungsträger-Gehäusehülse 10 in einen zugewandten Endabschnitt des Atemluft-Schlauchabschnitts 3 in einem Einsteckschritt 36 eingesteckt.

Figur 5 zeigt links oben eine entsprechend in den Atemluft-Schlauchabschnitt 3 eingesteckte innere Leitungsträger-Gehäusehülse 10. Im Rahmen des Einsteckschritts 36 werden die Kontaktpads 15, 16 der Leitungskomponenten 12, 13 zu den freigelegten Endabschnitten der Mantel-Drähte 5 bis 7 hin positioniert und ausgerichtet.

In einem nachfolgenden Verbindungsschritt 37 werden die Leitungskomponenten 12, 13 der Leitungsverbindung 14 beziehungsweise 27 mit den Endabschnitten der Mantel-Drähte 5 bis 7 elektrisch kontaktiert. Dies erfolgt mittels Ultraschall-Schweißen mit einer entsprechenden Ultraschall-Schweißeinrichtung 38.

Anschließend wird in einem Einlegeschritt 39 die vorgefertigte Roh-Baugruppe mit der inneren Leitungsträger-Gehäusehülse 10 und dem hiermit mechanisch und elektrisch verbundenen Atemluft-Schlauchabschnitt 3 in ein weiteres Spritzgusswerkzeug eingelegt. In einem anschließenden weiteren Umspritzschritt 40 wird dann die innere Leitungsträger-Gehäusehülse 10 sowie ein hieran angrenzender Endbereich des Atemluft-Schlauchabschnitts 3 zur Formung des äußeren Konnektorgehäuses 11 umspritzt. Hierbei wird auch die Sensoraufnahme 19 im äußeren Konnektorgehäuse geformt. Durch das Umspritzen im weiteren Umspritzschritt 40 ergibt sich die Integration der Luftführungs-Konnektorkomponente 9 mit dem Atemluft-Schlauchabschnitt 3, sodass die Luftführungs-Konnektorkomponente 9 des hergestellten Konnektors 8 einen integralen Bestandteil mit dem Atemluft-Schlauchabschnitt bildet.

Bei der Elektronikkomponente, die in der Komponenten- beziehungsweise Sensoraufnahme 19 aufgenommen werden kann, kann es sich allgemein um eine Sensoreinrichtung handeln, also beispielsweise um die Temperatursensoreinrichtung 2.

Nach dem weiteren Umspritzschritt 40 erfolgt in einem Kontrollschritt 41 eine optische Qualitätskontrolle 42 und eine elektrische Qualitätskontrolle 43, wobei die Baugruppe 1 einer Sichtprüfung sowie einer Überprüfung durch entsprechende optische und/oder elektrische/elektronische Messeinheiten unterzogen werden kann.

Anhand der Figuren 6 und 7 wird nachfolgend eine weitere Ausführung 45 der Baugruppe für das Patienten-Beatmungssystem beschrieben, die anstelle der Baugruppe zum Einsatz kommen kann, die vorstehend anhand der Figuren 1 bis 5 beschrieben wurde. Komponenten und Funktionen, die denjenigen entsprechen, die vorstehend in Bezugnahme auf die Figuren 1 bis 5 bereits erläutert wurden, tragen die gleichen Bezugsziffern und werden nicht nochmals im Einzelnen diskutiert.

Bei der Baugruppe 45 ist eine Leitungsverbindung 46, die ansonsten der 3D-Leitungsverbindung 27 der Ausführung nach den Figuren 1 bis 5 entspricht, zweidimensional ausgeführt. Die Kontaktpins 12, 13 dieser Leitungsverbindung sowie die Kontaktpads 15,16 liegen in einer gemeinsamen Anordnungsebene der 2D-Leitungsverbindung 46.

Figur 6 zeigt zudem einen Kern 47 eines Ultraschallschweißwerkzeuges zum Ultraschallschweißen der Leitungskomponenten 12, 13 der Leitungsverbindung 14 beziehungsweise 27 mit den Endabschnitten der Mantel-Drähte 5 bis 7 der Baugruppe 45.

Das Herstellungsverfahren der Baugruppe 45 entspricht grundsätzlich demjenigen, das vorstehend anhand der Figuren 4 und 5 bereits erläutert wurde.

Anstelle einer Vorformung einer 3D-Leitungsverbindung erfolgt beim Herstellen der Baugruppe 45 zunächst eine Bereitstellung der 2D-Leitungsverbindung 46 ausgehend von der als Leadframe ausgeführten 2D-Leiterstruktur 28. Dieses Bereitstellen kann ausschließlich durch Trennen der zugehörigen Leitungskomponenten von umgebenden Trag- beziehungsweise Leitungskomponenten des Leadframes 28 geschehen.

Ergebnis dieser Bereitstellung der 2D-Leitungsverbindungen 46 ist eine Mehrzahl derartiger 2D-Leitungsverbindungen 46, zum Beispiel drei derartige 2D-Leitungsverbindungen 46, die einer entsprechenden Mehrzahl der später anzuformenden inneren Leitungsträger-Gehäusehülsen 10 des jeweiligen Konnektors 8 der Baugruppe 45 zugeordnet sind. Diese mehreren 2D-Leitungsverbindungen 46 sind nach dem Bereitstellungsschritt zunächst noch mechanisch über Sollbruchstellen miteinander verbunden, wie vorstehend anhand der 3D-Leitungsverbindungen 27 der Ausführung nach den Figuren 1 bis 4 bereits erläutert.

Es erfolgt dann bei der Herstellung der Baugruppe 45 das Einsetzen 32, das Umspritzen, das Trennen 34, das Einstecken der Anschlusshülse 35 einschließlich des Positionierens und Ausrichtens der Kontaktpads 15, 16 zu den beigelegten Endabschnitten der Mantel-Drähte 5 bis 7, das elektrische Verbinden 37, das Einlegen 39, das weitere Umspritzen 40 sowie die Kontrolle 41 entsprechend dem, was vorstehend im Zusammenhang mit der Herstellung der Baugruppe 1 nach den Figuren 1 bis 5 bereits erläutert wurde.

## Patentansprüche

1. Verfahren zur Herstellung einer Baugruppe (1; 45) für ein Patienten-Beatmungssystem,
- mit einem Atemluft-Schlauchabschnitt (3) zur Führung von Beatmungsluft von einer Atemluftquelle hin zu einem Patienten, wobei längs eines Schlauchmantels (4) des Atemluft-Schlauchabschnitts (3) elektrisch leitfähige Mantel-Drähte (5 bis 7) geführt sind,
- mit einem mit dem Atemluft-Schlauchabschnitt (3) verbundenen Konnektor (8) zum Verbinden des Atemluft-Schlauchabschnitts (3) mit einer weiteren, die Beatmungsluft führenden Komponente des Patienten-Beatmungssystems, wobei eine Luftführungs-Konnektorkomponente (9) des Konnektors (8) einen integralen Bestandteil mit dem Atemluft-Schlauchabschnitt (3) bildet,
- wobei der Konnektor (8) eine Komponentenaufnahme (19) zum Einsetzen mindestens einer Elektronikkomponente (2) in den Konnektor (8) aufweist,
- wobei die Komponentenaufnahme (19) mindestens zwei Kontaktpins (12, 13) zum elektrischen Konnektieren mit der Elektronickomponente (2) aufweist,
mit folgenden Schritten:
- Bereitstellung mindestens einer Leitungsverbindung (27) zwischen den Kontaktpins (12, 13) der Komponentenaufnahme (19) und den Mantel-Drähten (5 bis 7),
- Umspritzen (33) der Leitungsverbindung (27) zur Formung einer inneren Leitungsträger-Gehäusehülse (10) des Konnektors (8),
- elektrisches Verbinden (37) der Leitungsverbindung (27) mit den Mantel-Drähten (5 bis 7),
- Umspritzen (40) der inneren Leitungsträger-Gehäusehülse (10) zur Formung eines äußeren Konnektorgehäuses (11) des Konnektors (8), in dem die Komponentenaufnahme (19) ausgeführt ist,
wobei beim Bereitstellen ein Vorformen (29) der Leitungsverbindung ausgehend von einer 2D-Leiterstruktur (28) erfolgt,
**dadurch gekennzeichnet,**
**dass** nach dem Vorformen (29) eine Mehrzahl von Leitungsverbindungen (27), die einer entsprechenden Mehrzahl von inneren Leitungsträger-Gehäusehülsen (10) zugeordnet werden, zunächst mechanisch miteinander verbunden bleiben,
- wobei beim Umspritzen (33) der Leitungsverbindungen (27) gleichzeitig diese Mehrzahl von Leitungsverbindungen (27) zur Formung der entsprechenden Mehrzahl von inneren Leitungsträger-Gehäusehülsen (10) in einer entsprechenden Mehrzahl von Kavitäten eines Spritzgusswerkzeugs (31) umspritzt werden,
- wobei nach dem Umspritzen (33) zur Formung der inneren Leitungsträger-Gehäusehülsen (10) eine mechanische Trennung der miteinander verbundenen Leitungsverbindungen (27) und damit eine Vereinzelung der inneren Leitungsträger-Gehäusehülsen (10) erfolgt.

2. Verfahren nach Anspruch 1, wobei beim Vorformen (29) ausgehend von der 2D-Leiterstruktur (28) eine 3D-Leitungsverbindung geformt wird.

3. Verfahren nach einem der Ansprüche 1 bis 2, wobei das elektrische Verbinden (37) durch Ultraschall-Schweißen erfolgt.

## Claims

1. A method for the manufacture of an assembly (1; 45) for a patient ventilation system,
- with a breathing air tube portion (3) for guiding breathing air from a breathing air source to a patient, wherein electrically conductive sheath wires (5 to 7) are guided along a tube sheath (4) of the breathing air tube portion (3),
- with a connector (8) connected to the breathing air tube portion (3) for connecting the breathing air tube portion (3) to a further component of the patient ventilation system carrying the breathing air, wherein an air conduction connector component (9) of the connector (8) forms an integral component with the breathing air tube portion (3),
- wherein the connector (8) comprises a component receptacle (19) for inserting at least one electronic component (2) into the connector (8),
- wherein the component receptacle (19) comprises at least two contact pins (12, 13) for electrically connecting with the electronic component (2),
comprising the following steps:
- providing at least one conducting connection (27) between the contact pins (12, 13) of the component receptacle (19) and the sheath wires (5 to 7),
- overmolding (33) of the conducting connection (27) to form an inner conduction carrier housing sleeve (10) of the connector (8),
- electrically connecting (37) the conducting connection (27) to the sheath wires (5 to 7),
- overmolding (40) of the inner conduction carrier housing sleeve (10) to form an outer connector housing (11) of the connector (8), in which housing the component receptacle (19) is implemented,
wherein pre-shaping (29) of the conducting connection on the basis of a 2D conductor structure (28) takes place during the providing step,
**characterized in that**
after pre-shaping (29), a plurality of conducting connections (27), which are associated with a corresponding plurality of inner conduction carrier housing sleeves (10), initially remain mechanically connected to each other,
- wherein during the overmolding (33) of the conducting connections (27), said plurality of conducting connections (27) are simultaneously overmolded to form the corresponding plurality of inner conduction carrier housing sleeves (10) in a corresponding plurality of cavities of an injection mold (31),
- wherein after the overmolding (33) for shaping the inner conduction carrier housing sleeves (10), a mechanical separation of the interconnected conducting connections (27) and thus a separation of the inner conduction carrier housing sleeves (10) takes place.

2. The method according to claim 1, wherein during pre-shaping (29) a 3D conducting connection is formed on the basis of the 2D conductor structure (28).

3. The method according to claim 1, wherein the electrical connection (37) is performed by ultrasonic welding.

## Revendications

1. Procédé de fabrication d'un ensemble (1 ; 45) pour un système de ventilation de patient,
- avec une section de tuyau d'air respirable (3) pour acheminer de l'air de ventilation d'une source d'air respirable à un patient, dans laquelle des fils de gaine (5 à 7) électriquement conducteurs sont guidés le long d'une gaine de tuyau (4) de la section de tuyau d'air respirable (3),
- avec un connecteur (8) relié à la section de tuyau d'air respirable (3) pour relier la section de tuyau d'air respirable (3) à un autre composant, conduisant l'air de ventilation, du système de ventilation de patient, dans lequel un composant de conduite d'air (9) du connecteur (8) fait partie intégrante de la section de tuyau d'air respirable (3),
- dans lequel le connecteur (8) présente un logement de composant (19) pour l'insertion d'au moins un composant électronique (2) dans le connecteur (8),
- dans lequel le logement de composant (19) présente au moins deux broches de contact (12, 13) pour la connexion électrique avec le composant électronique (2),
présentant les étapes suivantes :
- mise à disposition d'au moins une liaison filaire (27) entre les broches de contact (12, 13) du logement de composant (19) et les fils de gaine (5 à 7),
- surmoulage (33) de la liaison filaire (27) pour former une douille de boîtier de porte-conducteur interne (10) du connecteur (8),
- raccordement électrique (37) de la liaison filaire (27) aux fils de gaine (5 à 7),
- surmoulage (40) de la douille de boîtier de porte-conducteur interne (10) pour former un boîtier externe (11) du connecteur (8) dans lequel le logement de composant (19) est réalisé, un préformage (29) de la liaison filaire étant effectué lors de la mise à disposition, à partir d'une structure conductrice 2D (28),
**caractérisé en ce que**,
- après le préformage (29), une pluralité de liaisons filaires (27), qui sont affectées à une pluralité correspondante de douilles de boîtier de porte-conducteurs internes (10), restent, dans un premier temps, reliées les unes aux autres de manière mécanique,
- dans lequel, lors du surmoulage (33) des liaisons filaires (27), cette pluralité de liaisons filaires (27) est simultanément surmoulée pour former la pluralité correspondante de douilles de boîtier de porte-conducteurs internes (10) dans une pluralité correspondante de cavités d'un outil de moulage par injection (31),
- dans lequel, après le surmoulage (33) pour former les douilles de boîtier de porte-conducteurs internes (10), une séparation mécanique des liaisons filaires (27) reliées entre elles et donc une individualisation des douilles de boîtier de porte-conducteurs internes (10) est effectuée.

2. Procédé selon la revendication 1, dans lequel, lors du préformage (29), une liaison filaire 3D est formée à partir de la structure conductrice 2D (28).

3. Procédé selon l'une quelconque des revendications 1 à 2, dans lequel le raccordement électrique (37) est réalisé par soudage par ultrasons.
